(19) Europäisches Patentamt
European Patent Office
Office européen des brevets

(11) Publication number: **0 018 100**
**B1**

(12) **EUROPEAN PATENT SPECIFICATION**

(45) Date of publication of patent specification: **18.01.84**

(21) Application number: **80300915.8**

(22) Date of filing: **24.03.80**

(51) Int. Cl.³: **A 01 N 47/48,**
A 01 N 43/40,
A 01 N 41/02,
C 07 C 161/02,
C 07 C 161/05,
C 07 D 213/82

# (54) Substituted aromatic biologically active compound, a process for its preparation and its use as biocide.

(30) Priority: **12.04.79 GB 7913067**

(43) Date of publication of application:
**29.10.80 Bulletin 80/22**

(45) Publication of the grant of the patent:
**18.01.84 Bulletin 84/3**

(84) Designated Contracting States:
**BE CH DE FR GB IT NL SE**

(56) References cited:
**DE - A - 2 806 864**
**GB - A - 1 454 777**
**US - A - 3 231 596**

**CHEMICAL ABSTRACTS, vol. 87, no. 4, 25th July 1977, page 12, no. 23904h, Columbus, Ohio, USA, N.N. VOZNESENSKAYA et al. "Synthesis and thermal cyclotransformations of poly(thiocyanamides) and model compounds"**

**DERWENT JAPANESE PATENTS REPORT, vol. S, no. 44, 14th December 1971, abstract no. 70584S, London, GB**

(73) Proprietor: **IMPERIAL CHEMICAL INDUSTRIES PLC**
**Imperial Chemical House Millbank**
**London SW1P 3JF (GB)**

(72) Inventor: **Buckley, Alan John**
**14 Brockway**
**Grasscroft Near Oldham Lancs. OL4 4EU (GB)**

(74) Representative: **Pugsley, Roger Graham et al,**
**IMPERIAL CHEMICAL INDUSTRIES PLC Legal Department: Patents PO Box 6**
**Welwyn Garden City Herts, AL7 1HD (GB)**

(56) References cited:
**CHEMICAL ABSTRACTS, vol. 77, no. 1, 3rd July 1972, page 458, no. 5209z, Columbus, Ohio, USA**

**Z. Naturforschung 29b, (1974), pp. 693-4**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 018 100 B1**

Courier Press, Leamington Spa, England.

Substituted aromatic biologically active compound, a process for its preparation and its use as biocide

This invention relates to a method for protecting a medium from the growth of bacteria and fungi and to materials having anti-bacterial and anti-fungal activity.

It is known from Japanese Patent No 46—37512 that certain optionally substituted benzamides have anti-bacterial activity in an agricultural environment especially against rice blight (or blast). Among the possible substituents mentioned is the thiocyanate group which may be in one or more positions on the benzene ring relative to the amide group and one compound specifically disclosed is 2,5-dithiocyanatobenzamide. Although this compound was apparently novel there is no indication in the patent of the method by which it can be prepared. Other thiocyanatobenzamides specifically disclosed in the patent are 4-thiocyanatobenzamide, 3,5-diamino-4-thiocyanatobenzamide and 2-chloro-4-thiocyanatobenzamide.

The compound 2-thiocyanato-N-phenylbenzamide is known from Vysokomol Soedin Ser B *19(4)* (1977) pp 285—8 (Chem Abs [1977] 87:23904h) as an intermediate in the thermal degradation of certain aromatic polyamides. A paper entitled "1,2-Benzisothiazolinone: Reaction with sulphite, cyanide and cyanate" by W. V. Farrar in Z. Naturforschung *29b* (1974) p 693 discloses the formula of the compound 2-thiocyanatobenzamide but states that this compound is not formed when 1,2-benzisothiazolinone is reacted sequentially with sodium bisulphite and sodium cyanide. It has now, however, been found that 2-thiocyanto-benzamides are formed by the sequential reaction of bisulphite and cyanide ions with 1,2-benzisothiazolinones and that these compounds have particularly good biological activity compared with the isomeric 3-thiocyanato- and 4-thiocyanatobenazmides.

According to the present invention there is provided a method for the protection of a medium from attack by micro-organisms which comprises incorporating there in a compound of the formula:

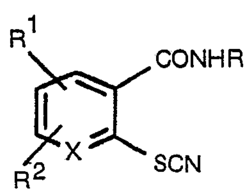

I

wherein

X is C or N;

R is H, $C_1$ to $C_{20}$ alkyl, cycloalky, or $C_3$ to $C_{20}$ alkenyl; and

$R^1$ and $R^2$ are each independently H, halogen, $C_1$ to $C_{16}$ alkyl or $C_1$ to $C_4$ alkoxy, or $R^1$ and $R^2$ together form a benzene ring fused to the ring to which they are both attached.

It is preferred that X is carbon.

Where R is alkyl or alkenyl it is preferably lower ($C_1$ to $C_4$) alkyl or lower ($C_3$ to $C_6$) alkenyl.

The alkyl and alkenyl group represented by R, $R^1$ and/or $R^2$ may be substituted, preferred substituents being hydroxy, alkoxy and halogen.

Examples of preferred lower alkyl and substituted lower alkyl groups are methyl, ethyl, n-propyl, isopropyl, n-butyl, 2-hydroxyethyl, 2-chloroethyl and 2-methoxyethyl. Examples of preferred lower alkenyl groups are propenyl and butenyl.

At least one of the alkyl groups represented by $R^1$ and $R^2$ may contain up to 16 carbon atoms in order to provide some lipophilicity. The alkoxy group represented by $R^1$ or $R^2$ is preferably lower ($C_1$ to $C_4$) alkoxy.

The quantity of the biologically active compound of Formula I which is used will depend upon the nature of the medium it is desired to protect and the micro-organisms to which the medium is susceptible. In general, the quantity is from 5 to 50,000 parts per million (ppm) by weight, based upon the weight of the medium being protected, and is more preferably from 50 to 10,000 ppm.

Where the medium is liquid, the compound may be added in the solid state but it is preferably added in the form of a solution in a suitable solvent, for example a lower alkanol, or a solvent/water mixture, or as a suspension or dispersion in water. Where an aqueous dispersion is used, this preferably includes a dispersing agent, for example the sodium salt of a nahthalene sulphonic acid/formaldehyde condensate, to assist in dispersing the compound evenly throughout the medium.

Solid media may be protected either by impregnation or superficial coating with the compound. Paint films may be protected by incorporation of the compound in the paint before it is applied to a surface.

The method according to the invention is particularly useful wherein the medium to be treated is a paint film or a water-based paint, e.g. an emulsion paint. Infection susceptible media, especially paint films and water-based paints protected against infection with micro-organisms by the incorporation therein of a biologically active compound according to Formula I, as hereinbefore defined, constitute a further feature of the present invention.

According to another feature of the invention there is provided the novel biologically active compound of Formula I wherein X, R, $R^1$ and $R^2$ are as hereinbefore defined provided that at least one of

R, $R^1$ and $R^2$ is a group other than H.

The compound of Formula I may be prepared from a compound of the formula

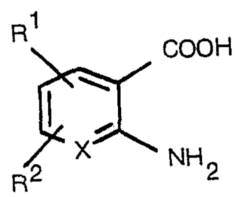

II

wherein X, $R^1$ and $R^2$ are as hereinbefore defined, by conversion of the amino group into a thiocyanato group and the carboxylic acid group into a carbamoyl group. These operations may be performed in either order. The intermediate thiocyanate compound of the formula:

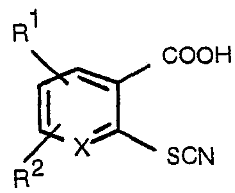

III

may be prepared by diazotisation of the amino group in the compound of Formula II followed by treatment of the diazonium compound with an alkali metal or ammonium thiocyanate. The compound of the Formula III may be converted in the compound of Formula I by treatment with a cholorinating agent, such as thionyl chloride or phosphorus pentachloride, to convert the —COOH group into a —COCl group, followed by reaction with an amine of the formula $RNH_2$, wherein R is as previously defined.

A preferred method for preparing the compound of Formula I, is by reaction of a compound containing an aromatic ring fused to a 1,2-isothioazolin-3-one ring or a substituted derivative thereof, such as 1,2-benzisothiazolin-3-one, with an alkali metal or ammonium bisulphite, to form a compound in which the aromatic ring carries a carbamoyl group and a thiosulphate group in ortho relationship, and conversion of the thiosulphate group into a thiocyanate group, by further reaction with an alkali metal or ammonium cyanide. Some of the intermediate thiosulphate compounds have significant biological activity, particularly against fungi.

A preferred thiosulphate compound has the formula:

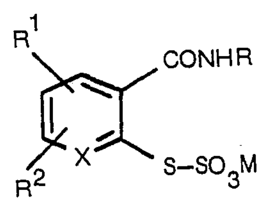

IV

wherein X, R, $R^1$ and $R^2$ have the previously defined meanings and M is an alkali metal or an ammonium ion.

The preferred thiosulphate compound may be prepared by reaction of a compound according to the formula:

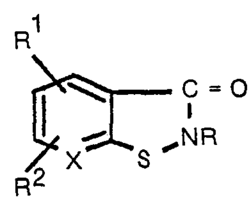

V

wherein X, R, $R^1$ and $R^2$ have the previously defined meanings, with an alkali metal or ammonium bisulphite in an aqueous or alcoholic medium at a temperature from 0°C to 100°C. The preferred thiosulphate compound may be converted into the equivalent thiocyanate compound by reaction with an alkali metal or ammonium cyanide at a temperature from 0°C to 100°C.

Example 1

Preparation of 2-thiocyanatobenzamide (2TB)

(a) *Preparation of Sodium 2-carbamoylbenzenethiosulphate*

75 parts sodium bisulphite liquor (40%), 16 parts benzisothiazolone and 25 parts water, are

stirred at 45—50°C for three (3) hours. The solution is filtered hot to remove impurities, cooled to room temperature; the precipitated product is filtered off and dried in the vacuum oven.

The crude product is recrystallised from ethanol, filtering hot to remove insoluble sodium sulphate, yielding 7 parts of a white crystalline solid product.

Elemental analysis of the product gave the following results (theoretical values in brackets): C 32.5 (32.0); H 2.4 (2.5); N 5.5 (5.5); S 24.8 (25.1); Na 8.8 (9.0); IR analysis showed stretches at 3200 $cm^{-1}$, 3380 $cm^{-1}$, 1675 $cm^{-1}$, 1415 $cm^{-1}$, 730 x 750 $cm^{-1}$ indicative of a $CONH_2$ group and an ortho disubstituted benzene ring.

(b) *Conversion of thiosulphate group to a thiocyanate group*

6.83 parts of the product of (a), 1.7 parts of potassium cyanide are both dissolved in the minimum amount of water. Solution of potassium cyanide is added to that of (I) with constant stirring. The white precipitate that is formed is filtered off and dried in the vacuum oven (3.2 parts).

Elemental analysis of the product gave the following results (theoretical values in brackets): C 53.6 (53.9); H 3.0 (3.4); N 15.8 (15.7); S 16.0 (18.0). IR analysis showed stretches at 3200—3450 $cm^{-1}$; 1675 and 1415 $cm^{-1}$; 2165 $cm^{-1}$ and 730—750 $cm^{-1}$ indicative of $—CONH_2$, —SCN and an ortho disubstituted benzene ring.

Example 2

Preparation of 5-chloro-2-thiocyanatobenzamide (5C2TB)

(a) *Preparation of 2-thiocyanato-5-chlorobenzoic acid*

11.2 parts of 5-chloroanthranilic acid, 2.61 parts of sodium hydroxide and 140 parts of water, are stirred to solution. 5.37 parts of sodium nitrite dissolved in 70 parts of water are added. The whole is cooled to <5°C. 42.3 parts of concentrated hydrochloric acid are added, with constant agitation. The temperature is held at <5°C throughout the acid addition and for a further 30 minutes. The product is a solution of diazo 5-chloroanthranilic acid. 18.87 parts of potassium thiocyanate with 7.92 parts of cuprous thiocyanate, in 280 parts of water, are heated to 70°C. The above diazo solution is then added, over a 30 minute period, maintaining a temperature of 70°C. The whole is stirred for a further 30 minutes at the same temperature, cooled to room temperature and filtered.

The filter cake is washed with 200 parts of water and pulled semi-dry at the pump. The filter cake is suspended in 220 parts of acetone, refluxed, filtered hot, to remove inorganic insolubles and cooled to room temperature. The solution is evaporated to 20 parts and cooled in a solid carbon dioxide/acetone bath, yielding 5.48 parts of a brown-yellow solid. Analysis of solid gave the following results:

(i) m.pt. 146—153°C

(ii) IR Spectrum broad band at 3250—2950 $cm^{-1}$ indicative of OH a sharp peak at 2170 $cm^{-1}$ indicative of SCN a sharp peak at 1680 $cm^{-1}$ C = O and peaks at 900 and 820 $cm^{-}$ indicative of a tri-substituted benzene ring.

(b) *Preparation of 2-thiocyanato-5-chlorobenzoyl chloride*

5.48 parts of the product of (a) and 56.8 parts of dry ether are charged to a reaction vessel, and 6.39 parts of phosphorus pentachloride are added slowly. The mixture is stirred at room temperature for 18 hours, the ether is distilled off under high vacuum at <30°C, and the vacuum (0.6 mm Hg) is maintained for one hour.

The resulting solid is extracted with 177.5 parts of dry ether, filtered to remove insoluble impurities, and cooled in solid carbon dioxide/acetone bath, until crystallisation commences. The crystals are filtered, dried over phosphorus pentoxide, in a dessicator, to yield 3.21 parts of a product having a melting point at 93—94°C.

(c) *Replacement of chlorine atom by amino group*

0.83 parts of product (b) and 42.6 parts of dry ether are stirred and cooled in solid carbon dioxide/acetone bath. 9.607 parts of ammonia dissolved in 7.1 parts dry ether is added in portions over a ten minute period. The whole is stirred with the above cooling for one hour and for a further hour at room temperature.

The resulting suspension is filtered and the filter cake washed with (2 x 7.0 parts) ether. The ether extracts are combined and evaporated to dryness to give a white solid which is recrystallised twice from ethanol to yield a white crystalline product, (5-chloro-2-thiocyanato benzamide). The results of analysis are:

(i) m.p. 239—241°C

(ii) elemental analysis (theoretical values in brackets): C 45.0 (45.2); H 2.3 (2.3); N 12.6 (13.2); S 14.7 (15.0)

(iii) IR Spectrum
Peaks at 3200—3450 $cm^{-1}$, 1670 and 1415 $cm^{-1}$ indicative of $CONH_2$ and at 2150 $cm^{-1}$ indicative of SCN.

Example 3

Preparation of 2-thiocyanato-N-methylbenzamide (2TNMB)

(a) *Preparation of 2-(methylcarbamoyl)benzene thiosulphate*

10 parts of N-methylbenzisothiazolone are added to 60 parts sodium bisulphite. The temperature is raised to 50°C, with constant stirring, at which point all the benzisothiazolone is dissolved, the whole is maintained at 50°C for 15 minutes, cooled to 0°C and evaporated to dryness.

The resulting solid is extracted twice with 100 parts of boiling methanol, filtered hot to remove inorganic impurities and evaporated to dryness to yield 15 parts of a white solid.

The IR spectrum showed bands at 3300 $cm^{-1}$, 3100 $cm^{-1}$, 1640 $cm^{-1}$ and 1550 $cm^{-1}$ indicative of —CONH— and bands at 1210—1740 $cm^{-1}$ and 1030 $cm^{-1}$ indicative of $—SO_3Na$.

(b) *Conversion of thiosulphate group to a thiocyanate group*

2.69 parts of the product of (a) are dissolved in 50 parts of water and a solution of 0.65 parts of potassium cyanide in 20 parts of water is added dropwise. A white precipitate forms. This is filtered off and dried to give 0.65 parts of 2-thiocyanato-N-methyl benzamide.

The IR spectrum showed peaks at 3200—3400 $cm^{-1}$ indicative of CO—NH—, 2165 $cm^{-1}$ indicative of —SCN, and 730—750 $cm^{-1}$ indicative of an ortho disubstituted benzene ring.

Example 4

Preparation of 2-thiocyanato-N-ethylbenzamide (2TNEB)

(a) *Preparation of sodium 2-(ethylcarbamoyl)benzene thiosulphate*

18.1 parts of N-ethylbenzisothiazolone is suspended in 136 parts sodium bisulphite liquor (30%), the benzisothiazolone forming a top layer and the suspension is heated, with constant stirrings, at 32°C, when the reaction mass becomes homogeneous. The temperature is allowed to rise to 50°C, it is then cooled to 0°C.

The solution is evaporated to dryness, under vacuum, to yield a white solid which is extracted twice with 100 parts of boiling methanol, and evaporated to dryness to yield 35.2 parts of a white solid.

The IR spectrum showed bands at 3300, 3100, 1640 and 1550 $cm^{-1}$, indicative of —CONH— and at 1210, 1240 and 1030 $cm^{-1}$ indicative of $—SO_3Na$.

(b) *Conversion of thiosulphate group to thiocyanate group*

2.83 parts of the product (a) are dissolved in 20 parts of water, and 0.65 parts of potassium cyanide are dissolved in 10 parts of water. The potassium cyanide solution is added to the first solution with constant stirring. After a short time (~ 5 mins) an oil separated as a lower layer. Stirring is continued for a further 2 hours, after which the oil solidifies. The resultant white solid is filtered off and dried in a vacuum oven.

Elemental analysis gave the following results (theoretical values in brackets): C 59.0 (58.3); H 5.2 (4.9); N 10.1 (13.6); S 14.7 (15.5). The IR spectrum showed the same characteristic peaks as the product of Example 3.

Assessment of Samples

Assessment 1 — In-vitro Antifungal and Antibacterial Activity of 2TB (Ex 1), 5C2TB (Ex 2) and 2TNMB (Ex 3)

1% solutions of each of these biocides in dimethylformamide were added to molten malt agar and to nutrient agar media to give in each case a final concentration in the test medium of 100 ppm. The test media were poured into petri dishes and allowed to cool before inoculation with test micro organisms. Nutrient agar was inoculated with three different test bacteria and malt agar was inoculated with five test fungi by means of an AM 80 automatic inoculator.

The nutrient agar plates were examined for the presence or absence of bacterial growth after incubation at 37°C for 24 hours and the malt agar dishes were examined for the presence or absence of fungal growth after incubation at 25°C for 5 days. The results of the assessments are given in Table I.

TABLE I

| Biocide | Growth of: Bacteria | | | Fungi | | | | |
|---|---|---|---|---|---|---|---|---|
| | A | B | C | D | E | F | G | H |
| Control | + | + | + | + | + | + | + | + |
| 2TB | + | – | – | – | + | – | – | – |
| 5C2TB | + | – | – | * | + | – | – | – |
| 2TNMB | + | + | – | – | + | – | – | – |

*Notes on Table I*

(1) The micro organisms used in the tests are as follows:

A = Pseudomonas aeruginosa
B = Escherichia coli
C = Staphylococcus aureus
D = Pullularia pullulans
E = Aspergillus niger
F = Cladosporium sphaerospermum
G = Alternaria tenuis
H = Chaetomium globosum.

(2) + = Growth of micro organism
– = No growth of micro organism (i.e. total inhibition).

(3) * indicates that the compound was not tested against this micro-organism.

Assessment 2 — Activity of 2TB as an In-can Paint Preservative

2TB as a 1% solution in dimethylformamide was added to 50 g quantities of a styrene-acrylic emulsion paint to give final concentrations of 100 ppm and 200 ppm in the wet paint. The control contained no biocide but an equal amount of dimethylformamide to that present in the paint containing 200 ppm of biocide. Each paint was inoculated with 0.5 ml of a mixture of overnight broth cultures of 3 bacteria once per week for 2 weeks. Survivors were determined each week at 1 and 3 days after each inoculation by the decimal dilution method. The results of the assessment are given in Table II.

TABLE II

| Sample | Survivors Week 1 | | Week 2 | |
|---|---|---|---|---|
| | Day 1 | Day 3 | Day 1 | Day 3 |
| 200 ppm 2TB | 10 | 10 | 10 | 10 |
| 100 ppm 2TB | 10 | 10 | 10 | 10 |
| Control | $8.0 \times 10^6$ | $2.0 \times 10^7$ | $5.0 \times 10^6$ | $2.0 \times 10^7$ |

Notes on Table II

(1) The bacteria used in the assessment were A and B from Assessment 1 and Enterobacter cloacae.

The results demonstrate that, at a level of 100—200 ppm 2TB has a high activity as an in-can paint preservative.

Assessment 3 — Activity of 2TB as a Paint Film Fungicide

2TB was added to a PVA-copolymer emulsion paint to give a concentration of 3000 ppm in the wet paint. The paint had the following composition:

| | | |
|---|---|---|
| Tap water | 258 g | millbase 63.1% |
| Ethylene glycol | 10 g | |
| Cellosolve | 7.5 g | |
| Nopco NDW | 1.5 g | |
| Calgon PT (50% solution) | 2 g | |
| Cellofas B50 | 5.5 g | |
| China Clay Grade D | 67.5 g | |
| Tioxide RCR | 280 g | |
| Total | 632 g | |

PVA copolymer emulsion (ICI x 935—6) 36.8%
0.880 ammonia 0.1%
0.880 ammonia 0.1%

0.3 g 2TB was added to 63 g millbase mixture and 50 g beads was added. The millbase-fungicide mixture was "red-devilled" for 35 minutes and 37 g of latex was added slowly, with hand stirring. The beads were sieved out of the emulsion paint and the latter was transferred into glass bottle which were tightly sealed and stored at 50°C for 5 weeks.

The control paint contained no added fungicide.

12" x 6" x $\frac{1}{2}$" Cedar panels were sprayed with one coat of Dulux (Dulux is a registered trade mark belonging to Imperial Chemical Industries PLC) white wood primer and two coats of Dulux Brilliant White undercoat. Two coats of the emulsion paint was applied, by brush, to one side of a panel, and 24 hours were allowed between each coat for drying.

The backs and edges of the panels were painted with one coat of aluminium paint in order to seal the surfaces not coated with the emulsion paint.

The painted panesl were exposed in a Malayan jungle site and were examined regularly for the development of mold growth. The extent of contamination was recorded as the % of the panel covered by the mould growth and the results are set out in Table III.

TABLE III

| Sample | Replicate Panels | % Area Moulded After Exposure Period of: Weeks 10 | 13 | 16 | 18 | 20 | 22 | 24 | 26 | 28 |
|---|---|---|---|---|---|---|---|---|---|---|
| 2TB, | 1 | 0 | 0 | 5 | 5 | 5 | 5 | 10 | 10 | 10 |
| 3000 ppm | 2 | 0 | 0 | 5 | 5 | 10 | 15 | 15 | 20 | 20 |
| Control | 1 | 0 | 80 | 90 | 95 | 100 | — | — | — | — |
| | 2 | 0 | 85 | 95 | 100 | — | — | — | — | — |

The results indicate that 2TB possess marked paint film fungicide activity.

Assessment 4 — Antialgal Activity of 2TB

*Ankystrodesmus spirilliformis, Stichococcus bacillaris,* and *Chlamydomonas reinhardii* were inoculated into one-litre batches of Difco algae broth and incubated at 18°C for 2 weeks in an illuminated incubator. The three cultures were then combined and the mixed culture was dispensed in 50 ml volumes into 100 ml sterile conical flasks.

2TB was added to each flask to give final concentrations of 5.0 and 10.0 ppm. The control consisted of the same algal mixture without the addition of biocide.

The flasks were incubated up to 14 days in the illuminated incubator and were examined for the presence or absence of the green colouration of the algae cells. The retention of the green colour indicated that the algae remained viable, whereas discolouration of the solution indicated that the algae had been killed. The results are shown in Table IV.

TABLE IV

| Sample | Survival of Algae After | | |
|---|---|---|---|
| | 3 days | 7 days | 14 days |
| 2TB, 5.0 ppm | + | + | + |
| 2TB, 10.0 ppm | — | — | — |
| Control (no biocide) | + | + | + |

The results demonstrate that 2TB has algicidal activity.

Example 5

Preparation of 2-thiocyanato-N-t-butylbenzamide (2TNtBB)

(a) *Preparation of 2-(t-butylcarbamoyl)benzene thiosulphate*

8.28 parts of N-t-butylbenziosothiazolone are suspended in 80 parts of sodium bisulphite liquor (40%), the benzisothiazolone forming a top layer. The suspension is heated, with constant stirring till at 63°C the reaction mass becomes homogeneous. The temperature is held at 63°C for a further 30 minutes.

The solution is cooled to room temperature and 9.6 parts of a white solid crystallises out.

IR spectrum shows band at 3300, 3100, 1640, 1550 $cm^{-1}$ indicative of —CONH— and at 1210, 1240 and 1030 $cm^{-1}$ indicative of $—SO_3Na$.

(b) *Conversion of thiosulphate group to thiocyanate group*

2.82 parts of the product of (a) are suspended in 50 parts of water. This heated, with continuous stirring to 40°C, at which point the solid dissolves. Maintaining the above temperature, 0.65 part of potassium cyanide dissolved in 10 parts of water is added, an immediate precipitate is formed. The reaction mass is cooled to room temperature, the solid filtered off and dried in the vacuum oven.

Elemental analysis gives the following results (theoretical in brackets): C 61.5 (62.3); H 6.3 (4.8).

Example 6

Preparation of 2-thiocyanato-N-2-chloroethylbenzamide (2TN2CEB)

(a) *Preparation of 2-(2-chloroethylcarbamoyl)benzene thiosulphate*

2.14 Parts of N-2-chloroethylbenzisothiazolone are suspended in a solution of 4 parts of sodium bisulphite liquor (40%) and 50 parts of water. The reaction mass is heated with stirring to 50°C. At this temperature the majority of solid is dissolved, leaving trace impurities. The liquors are decanted off from the impurities and allowed to cool to room temperature.

As no solid precipitates from solution, the reaction mass is evaporated to dryness to yield a tacky solid. This is extracted with boiling ethanol, filtered at the boil to remove inorganics, cooled to room temperature and evaporated to dryness to yield 2.9 parts of a white powdery hydroscopic material.

(b) *Conversion of thiosulphate group to thiocyanate group*

1.4 parts of the product of (a) are dissolved in 20 parts of water and 0.286 parts of potassium cyanide are dissolved in 10 parts of water. The potassium cyanide solution is added to the first solution with constant stirring. An immediate precipitate is formed. This is filtered off and dried at the pump.

Elemental analysis gives the following results: C 50.1 (49.0); H 3.7 (3.7); N 10.8 (11.6); Cl 13.3 (14.8); S 14.0 (13.3).

IR shows character similar to that of the product of Example 3.

Example 7

Preparation of 2-thiocyanato-N-2-hydroxyethylbenzamide (2TN2HEB)

(a) *Preparation of 2-(2-hydroxyethylcarbamoyl)benzene thiosulphate*

Procedure as for Example 6 using 3.0 parts of N-2-hydroxyethylbenzisothiazolone and 40 parts of sodium bisulphite liquor (40%). Reaction mass heated to 50°C, cooled. As no solid precipitates out, solution is evaporated dry to yield a white solid which is extracted with boiling ethanol, filtered hot to remove inorganics, cooled, and evaporated dry to yield 4.5 parts of a white powdery solid.

IR spectrum shows bands typical of thiosulphates.

(b) *Conversion of thiosulphate group to thiocyanate group*

1.5 parts of the product from (a) are dissolved in 5 parts of water. 0.32 part of potassium cyanide are dissolved in 2 parts of water. The potassium cyanide solution is added to that of the first solution with constant stirring. An immediate precipitate is formed. This is filtered off and dried in the vacuum oven.

Elemental analysis gives the following results: C 52.5 (54.1); H 4.4 (4.5); N 11.8 (12.6); S 13.9 (14.4).

IR spectrum shows same characteristic peaks as Example 3.

Example 8

Preparation of 2-thiocyanato-N-allylbenzamide (2TNAB)

(a) *Preparation of 2-(allylcarbamoyl)benzene thiosulphate*

5.2 parts of N-allylbenzisothiazolone are suspended in a solution of 9 parts of sodium bisulphite liquor (40%) and 50 parts of water. The reaction mass is heated with stirring to 50°C, at which point all solid is in solution, cooled to room temperature, and as no solid precipitates out, evaporated to dryness to yield a yellow solid. This solid is extracted with boiling ethanol, filtered hot to remove inorganics, cooled and evaporated dry to yield 5.1 parts of a yellow solid.

IR spectrum shows bands typical of thiosulphates.

(b) *Conversion of thiosulphate group to thiocyanate group*

1.47 parts of the product from (a) are dissolved in 10 parts of water. 0.325 part of potassium chloride are dissolved in 5 parts of water. The solution of the product from (a) is cooled in an ice bath, and with constant stirring the solution of potassium cyanide is added to it. An immediate yellow solid is formed.

The yellow solid suspended in water is extracted with 10 parts of chloroform, which is separated off from the upper aqueous layer, dried over anhydrous magnesium sulphate, filtered and evaporated to yield the required product.

The IR spectrum shows the same characteristic peaks as the product of Example 3.

Example 9

Preparation of 2-thiocyanato-N-neopentylbenzamide (2TNNPB)

(a) *Preparation of 2-(neopentylcarbamoyl)benzene thiosulphate*

4.4 parts of N-neopentylbenzisothiazolone are suspended in a solution of 5 parts sodium bisulphite liquor (40%) and 50 parts of water. The reaction mass is heated with stirring to 90°C. At this temperature the solution is a pale yellow, and a brown oil has formed on the walls of the reaction vessel.

The solution is decanted off from the oil, cooled to room temperature and evaporated dry to yield a yellow solid which was extracted with boiling ethanol, filtered hot to remove inorganics, cooled and evaporated to dryness to yield 3.65 parts of yellow solid. The IR spectrum shows bands typical of thiosulphates.

(b) *Conversion of thiosulphate group to thiocyanate group*

3.2 parts of the product from (a) are dissolved in 10 parts of water. 0.65 part of potassium cyanide are dissolved in 10 parts of water, with constant stirring, the solution of cyanide is added to the solution of (a). A solid is precipitated immediately; this is filtered off and dried in vacuum oven.

Elemental analysis shows: C 65.7 (67.2); H 7.1 (6.7); N 11.1 (12.0); S 13.1 (13.7).

The IR spectrum shows same characteristic peak as the product of Example 3.

Example 10

Preparation of 4-chloro-2-thiocyanatobenzamide (4C2TB)

10.4 parts of anhydrous ammonia is dissolved in 50 parts of dried tetrahydrofuran held at −60°C by means of a cooling bath. 4.6 parts of 4-chloro-2-thiocyanato benzoic acid chloride in 50 parts of tetrahydrofuran is added slowly dropwise to the ammonia solution at −60°C, and the mixture is allowed to rise to ambient temperature before standing for 12 hours. The resultant suspension is filtered to remove inorganic material, the filtrates reduced to dryness, and the residue purified by fractional recrystallisation from ethanol to give 0.8 g of 4-chloro-2-thiocyanatobenzamide m.p. 224—226°C. The IR spectrum shows the characteristic —SCN absorption at 2160 $cm^{-1}$.

Example 11

Preparation of 3-carbamoyl-2-thiocyanatopyridine (3C2TP)

4.19 parts of 2-chloronicotinamide, 4.55 parts of potassium thiocyanate and 43 parts of glacial acetic acid are stirred at 40—44°C for 24 hours under an atmosphere of nitrogen. The solution is poured onto 200 g crushed ice and the product filtered, washed with water and dried. Yield 0.63 g, m.pt. 179—181°C. A small portion when re-crystallised from aqueous acetone yields pale yellow crystals, m.pt. 192—194°C. The IR spectrum contains a thiocyanate band at 2160 $cm^{-1}$.

| | | | | |
|---|---|---|---|---|
| Analysis: Found: | C 47.0; | H 2.6; | N 22.9; | S 17.0. |
| Calculated for $C_7H_5N_3OS$: | C 46.9; | H 2.8; | N 23.5; | S 17.9. |

Example 12

Preparation of 3-thiocyanato-2-naphthoic acid amide (3T2NAA)

3.84 parts of anhydrous ammonia is dissolved in 50 parts of dry ether held at −60°C by means of a cooling bath. 1.81 parts of 3-thiocyanato-2-naphthoic acid chloride in 50 parts of dry ether is added dropwise over a period of ten minutes. An exotherm of 15° is observed before the reaction mixture is stirred at −60°C for 1 hour followed by 25 hours at ambient temperature. The suspension is filtered to remove ammonium chloride, the ether filtrates reduced to dryness and the product purified by fractional crystallisation from ethanol to give 0.44 g of 3-thiocyanato-2-naphthoic acid amide m.p. 176—178°C. The IR spectrum shows the characteristic —SCN absorption at 2150 $cm^{-1}$.

Example 13

Preparation of 2-thiocyanato-N-cyclohexylbenzamide (2TNCHB)

(a) *Preparation of sodium 2-(cyclohexylcarbamoyl)benzene thiosulphate (2NCHCBTS)*

N-cyclohexylbenzisothiazolone (3.3 parts) is distilled in methanol (25 parts). To this solution, whilst stirring, is added sodium bisulphite liquor, 40%, (28 parts); an exotherm of 8°C occurs (i.e. 21 29°C). The temperature is raised to 40°C, to ensure complete reaction, cooled to room temperature, filtered, and evaporated to dryness. The resultant solid is extracted with methanol, inorganic material filtered off, and the methanol solution evaporated to yield a light brown solid sodium 2-(cyclohexylcarbamoyl)benzene thiosulphate (3.8 parts).

The IR spectrum has bands at 3370, 3060, 1620, 1530 $cm^{-1}$ arising from the —CONH— group and bands at 1210 and 1030 $cm^{-1}$ arising from the $—SO_3Na$ group.

(b) *Conversion of thiosulphate group to a thiocyanate group*

The sodium 2-(cyclohexylcarbamoyl)benzene thiosulphate (3.8 parts) is dissolved in water (50 parts) and methanol (10 parts). To this solution is added potassium cyanide (0.715 parts) dissolved in water (10 parts). The white precipitate formed is filtered off and dried in a vacuum oven, to yield a white powder 2-thiocyanato-N-cyclohexylbenzamide (2.1 g). The IR spectrum has a peak at 2160 $cm^{-1}$ due to the —SCN group.

Example 14

Preparation of 5-methyl-2-thiocyanatobenzamide (5M2TB)

(a) *Preparation of sodium 2-carbamoyl-5-methylbenzene thiosulphate (2C4MBTS)*

5-methylbenzisothiazolone (2.05 parts) is suspended in sodium bisulphite liquor, 40%, (24 parts). The temperature is raised, with constant stirring to 60°C at which point the benzisothiazolone has dissolved, leaving a small quantity of undissolved impurity. The solution is filtered hot to remove the impurity and cooled to room temperature. A white solid precipitates out. This is filtered, extracted with boiling methanol, filtered hot, and evaporated to dryness to yield a white solid, sodium 2-carbamoyl-5-methylbenzene thiosulphate (3.3 g).

The IR spectrum shows the same characteristic peaks and bands as the product of Example 13(a).

(b) *Conversion of thiosulphate group to thiocyanate group*

The sodium 2-carbamoyl-5-methylbenzene thiosulphate (2.5 parts) is dissolved in water (25 parts). To this solution is added potassium cyanide (0.58 parts). The white precipitate formed is filtered off and dried in vacuum oven, yielding a white powder, 2-thiocyanato-5-methylbenzamide (0.8 parts).

The IR spectrum shows the same characteristic peaks associated with the —SCN group as the product of Example 13.

Assessment 5 — In-vitro Antifungal and Antibacterial Activity of the products of Examples 4 to 14

The products of Examples 4 to 14 are assessed according to the procedure described in Assessment 1 and the results are displayed in Table V.

TABLE V

| Biocide | Growth of | | | | | | | |
|---|---|---|---|---|---|---|---|---|
| | Bacteria | | Fungi | | | | | |
| | A | B | C | D | E | F | G | H |
| Control | + | + | + | + | + | + | + | + |
| 2TNEB | + | + | — | * | — | — | + | * |
| 2TNEB | + | + | — | * | — | — | + | * |
| 2TNtBB | — | + | — | — | — | — | — | — |
| 2TN2CEB | + | + | — | — | — | — | * | — |
| 2TN2HEB | + | — | — | — | + | — | — | + |
| 2TNAB | + | — | — | — | — | — | — | — |
| 2TNNPB | + | + | — | — | — | — | — | — |
| 4C2TB | + | — | — | — | + | — | — | — |
| 3C2TP | + | — | — | — | — | — | + | + |
| 3T2NAA | + | — | — | — | — | — | — | — |
| 2TNCHB | + | + | — | — | — | — | * | — |
| 5M2TB | — | — | — | — | — | — | * | — |

*Notes*
(1) The letters A to H have the meanings defined in Assessment 1.
(2) * indicates that compound was not tested against this fungus

**Claims**

1. A method for the protection of a medium from attack by micro-organisms which comprises incorporating into the medium a biologically active compound having the formula:

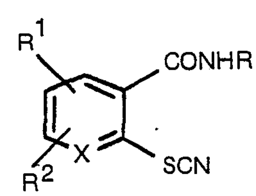

I

wherein

X is C or N;

R is H, $C_1$ to $C_{20}$ alkyl, cycloalkyl, or $C_3$ to $C_{20}$ alkenyl; and

$R^1$ and $R^2$ are each independently H, halogen, $C_1$ to $C_{16}$ alkyl or $C_1$ to $C_4$ alkoxy, or $R^1$ and $R^2$ together form a benzene ring fused to the ring to which they are both attached.

2. A method according to Claim 1 wherein, in the compound of Formula I, the alkyl or alkenyl group represented by R is substituted by a group selected from hydroxy, alkoxy and halogen.

3. A method according to Claim 1 or Claim 2 wherein, in the compound of Formula I, the alkyl group represented by $R^1$ or $R^2$ is substituted by a group selected from hydroxy, alkoxy and halogen.

4. A method according to Claim 1 wherein, in the compound of Formula I, X represents carbon and each of R, $R^1$ and $R^2$ represents hydrogen.

5. A medium which is susceptible to attack by micro-organisms containing from 5 to 50,000 parts per million, by weight, of the compound of Formula I as defined in any one of Claims 1 to 4.

6. A compound of the formula:

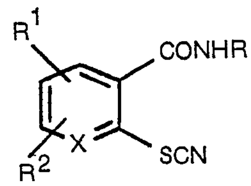

I

wherein

X is C or N;

R is H, $C_1$ to $C_{20}$ alkyl, cycloalkyl, or $C_3$ to $C_{20}$ alkenyl; and

$R^1$ and $R^2$ are each independently H, halogen, $C_1$ to $C_{16}$ alkyl or $C_1$ to $C_4$ alkoxy, or $R^1$ and $R^2$ together form a benzene ring fused to the ring to which they are both attached. Provided that at least one of R, $R^1$ and $R^2$ is a group other than H.

7. A compound according to Claim 6 wherein the alkyl or alkenyl group represented by R is substituted by a group selected from hydroxy, alkoxy and halogen.

8. A compound according to Claim 6 or Claim 7 wherein the alkyl group represented by $R^1$ or $R^2$ is substituted by a group selected from hydroxy, alkoxy and halogen.

**Patentansprüche**

1. Verfahren zum Schützen eines Mediums gegen den Angriff durch Mikroorganismen, bei welchem in das Medium eine biologisch aktive Verbindung der Formel eingearbeitet wird:

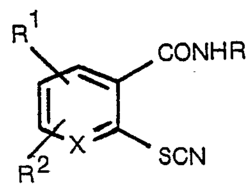

I

worin

X für C oder N steht,

R für H, $C_1$—$C_{20}$-Alkyl, Cycloalkyl oder $C_3$—$C_{20}$-Alkenyl steht und

$R^1$ und $R^2$ jeweils unabhängig voneinander für H, Halogen, $C_1$—$C_{16}$-Alkyl oder $C_1$—$C_4$-Alkoxy stehen oder gemeinsam einen Benzolring bilden, der mit dem Ring kondensiert ist, an den sie beide gebunden sind.

2. Verfahren nach Anspruch 1, bei welchem in der Verbindung der Formel I die durch R dargestellte Alkyl- oder Alkenylgruppe durch eine aus Hydroxy, Alkoxy und Halogen ausgewählte Gruppe substituiert ist.

3. Verfahren nach Anspruch 1 oder 2, bei welchem in der Verbindung der Formel I die durch $R^1$ oder $R^2$ dargestellte Alkylgruppe durch eine aus Hydroxy, Alkoxy und Halogen ausgewählte Gruppe substituiert ist.

4. Verfahren nach Anspruch 1, bei welchem in der Verbindung der Formel I X für Kohlenstoff steht und R, $R^1$ und $R^2$ jeweils für Wasserstoff stehen.

5. Medium, welches dem Angriff durch Mikroorganismen zugänglich ist und welches 5 bis 50000 Gew.-Teile je Million der Verbindung der Formel I nach einem der Ansprüche 1 bis 4 enthält.

6. Verbindung der Formel:

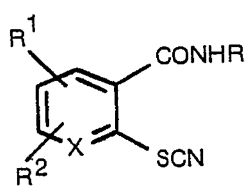

I

worin

X für C oder N steht,

R für H, $C_1$—$C_{20}$-Alkyl, Cycloalkyl oder $C_3$—$C_{20}$-Alkenyl steht und

$R^1$ und $R^2$ jeweils unabhängig voneinander für H, Halogen, $C_1$—$C_{16}$-Alkyl oder $C_1$—$C_4$-Alkoxy stehen oder gemeinsam einen Benzolring bilden, der mit dem Ring kondensiert ist, an den sie beide gebunden sind,

mit der Maßgabe, daß mindestens eines der Symbole R, $R^1$ und $R^2$ für eine andere Gruppe als H steht.

7. Verbindung nach Anspruch 6, bei welcher die durch R dargestellte Alkyl- oder Alkenylgruppe durch eine aus Hydroxy, Alkoxy und Halogen ausgewählte Gruppe substituiert ist.

8. Verbindung nach Anspruch 6 oder 7, bei welcher die durch $R^1$ oder $R^2$ dargestellte Alkylgruppe durch eine aus Hydroxy, Alkoxy und Halogen ausgewählte Gruppe substituiert ist.

**Revendications**

1. Procédé pour protéger un milieu de l'attaque par des micro-organismes, qui consiste à incorporer au milieu un composé biologiquement actif répondant à la formule:

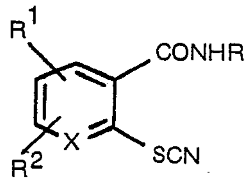

. I

dans laquelle X représente C ou N;

R représente H, un groupe alkyle en $C_1$ à $C_{20}$, cycloalkyle ou alcényle en $C_3$ à $C_{20}$; et

$R^1$ et $R^2$ représentent chacun, indépendamment, H, un halogène, un groupe alkyle en $C_1$ à $C_{16}$ ou un groupe alkoxy en $C_1$ à $C_4$, ou bien

$R^1$ et $R^2$ forment ensemble un noyau benzénique soudé au noyau auquel ils sont tous deux attachés.

2. Procédé suivant la revendication 1, dans lequel le groupe alkyle ou le groupe alcényle représentés par R dans le composé de formule I est substitué par un groupe choisi entre un groupe hydroxy, un groupe alkoxy et un halogène.

3. Procédé suivant la revendication 1 ou la revendication 2, dans lequel le groupe alkyle représenté par $R^1$ ou $R^2$ dans le composé de formule I est substitué par un groupe choisi entre un groupe hydroxy, alkoxy et un halogène.

4. Procédé suivant la revendication 1, dans lequel X représente le carbone et chacun de R, $R^1$ et $R^2$ représente l'hydrogène dans le composé de formule I.

5. Milieu exposé à l'attaque par des micro-organismes, contenant 5 à 50 000 ppm en poids de composé de formule I comme défini dans l'une quelconque des revendications 1 à 4.

6. Composé de formule

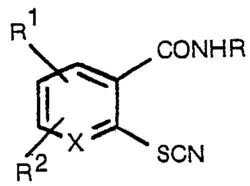

. I

dans laquelle

X représente C ou N;

R représente H, un groupe alkyle en $C_1$ à $C_{20}$, un groupe cycloalkyle ou un groupe alcényle en $C_3$ à $C_{20}$; et $R^1$ et $R^2$ représentent chacun indépendamment H, un halogène, un groupe alkyle en $C_1$ à $C_{16}$ ou un groupe alkoxy en $C_1$ à $C_4$, ou bien $R^1$ et $R^2$ forment ensemble un noyau benzénique soudé au noyau auquel ils sont tous deux attachés, sous réserve qu'au moins l'un de R, $R^1$ et $R^2$ soit un groupe autre que H.

7. Composé suivant la revendication 6, dans lequel le groupe alkyle ou alcényle représenté par R est substitué par un groupe choisi entre un groupe hydroxy, un groupe alkoxy et un halogène.

8. Composé suivant la revendication 6 ou la revendication 7, dans lequel le groupe alkyle représenté par $R^1$ ou $R^2$ est substitué par un groupe choisi entre un groupe hydroxy, un groupe alkoxy et un halogène.